# EUROPEAN PATENT APPLICATION

(11) **EP 4 293 006 A1**
(43) Date of publication of application: **20.12.2023**
(21) Application number: 22893219.0
(22) Date of filing: 10.11.2022
(51) Int. Cl.: C07C 22/08, C07C 17/02, C08F 212/14, C08F 212/36

(54) **COMPOUND, METHOD FOR PREPARING SAME, AND SINGLE MOLECULE AND POLYMER DERIVED FROM SAME**

(30) Priority: 11.11.2021 KR 20210154715
(71) Applicant: Lg Chem, Ltd., Seoul 07336 (KR)
(72) Inventor: JUNG, Jihye, Daejeon 34122 (KR); PARK, Beomsu, Daejeon 34122 (KR); KIM, Hyun Cheol, Daejeon 34122 (KR)
(74) Representative: Goddar, Heinz J.
(86) International application number: PCT/KR2022/017634
(87) International publication number: WO 2023/085800

(57) **Abstract**

The present specification relates to a compound of Chemical Formula 1, a method for preparing the same, and a single molecule and a polymer derived from the compound.

## Description

### [Technical Field]

This application claims priority to and the benefit of Korean Patent Application No. 10-2021-0154715 filed in the Korean Intellectual Property Office on November 11, 2021, the entire contents of which are incorporated herein by reference.

The present specification relates to a compound, a method for preparing the same, and a single molecule and a polymer derived from the compound.

### [Background Art]

Introduction of a perfluoalkyl group into an aromatic compound in the related art was induced through a nucleophilic aromatic substitution reaction using a Grignard reagent and halogenated benzene or derivatives thereof or a nucleophilic substitution reaction using a reaction in the opposite direction to that of the nucleophilic aromatic substitution reaction, but in such cases, development is difficult because there is a disadvantage in that a difficult-to-handle Grignard reagent needs to be used, a large amount of halogen compounds need to be used, and the reaction is sensitive to air and moisture.

### [Detailed Description of the Invention]

### [Technical Problem]

The present specification provides a compound, a method for preparing the same, and a single molecule and a polymer derived from the compound.

### [Technical Solution]

An exemplary embodiment of the present specification provides a compound of the following Chemical Formula 1.

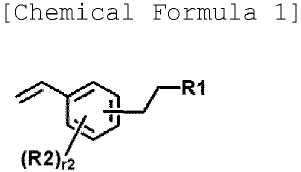

In Chemical Formula 1,
R1 is -CₙF₂ₙ₊₁,
n is an integer from 1 to 20,
R2 is hydrogen; deuterium; a halogen group; an ester group; or a substituted or unsubstituted straight-chained or branched alkyl group having 1 to 10 carbon atoms,
r2 is an integer from 1 to 4, and
when r2 is 2 or higher, two or more R2's are the same as or different from each other.

An exemplary embodiment of the present specification provides a method for preparing a compound of the following Chemical Formula 1, the method including: (s1) introducing the following Chemical Formula A, CₙF₂ₙ₊₁X, trialkyltin hydride and a solvent and stirring the resulting mixture under nitrogen gas; and (s2) adding a radical initiator thereto and stirring the resulting mixture.

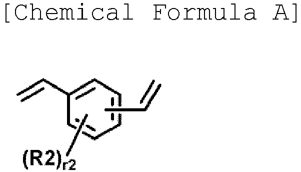

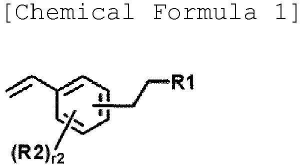

In Step (s1), Chemical Formula A and Chemical Formula 1,
R1 is -CₙF₂ₙ₊₁,
n is an integer from 1 to 20,
R2 is hydrogen; deuterium; a halogen group; an ester group; or a substituted or unsubstituted straight-chained or branched alkyl group having 1 to 10 carbon atoms,
r2 is an integer from 1 to 4,
when r2 is 2 or higher, two or more R2's are the same as or different from each other, and
X is a halogen group.

An exemplary embodiment of the present specification provides a mixture including two or more of the compounds.

Another exemplary embodiment of the present specification provides a single molecule derived from the compound.

Still another exemplary embodiment of the present specification provides a polymer including a monomer derived from the compound.

### [Advantageous Effects]

A compound according to an exemplary embodiment of the present specification, and a single molecule and a polymer derived therefrom are used as an electronic material, an organic insulating material, and/or a substrate material.

A method for preparing the compound according to an exemplary embodiment of the present specification has an advantage in that the compound is prepared under conditions where the reaction temperature is low. As a result, since the polymerization is suppressed to reduce side reactions, the yield of the compound of Chemical Formula 1, which is a target compound, is increased.

The compound according to an exemplary embodiment of the present invention can be used as a multifunctional monomer to exhibit the effects of achieving low refractive index, low relative permittivity, low surface energy and low dissipation factor.

### [Brief Description of Drawings]

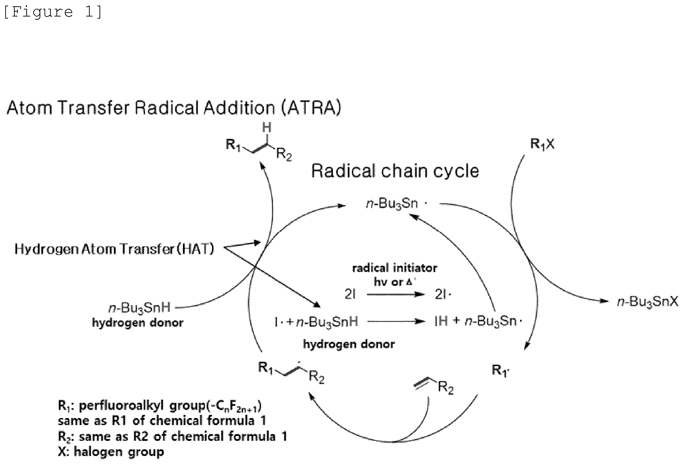

FIG. 1 is a view describing the mechanism of an atom transfer radical addition (ATRA) reaction.

### [Best Mode]

Hereinafter, the present specification will be described in more detail.

An exemplary embodiment of the present specification provides a compound of the following Chemical Formula 1.

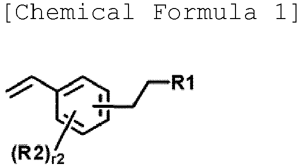

In Chemical Formula 1,
R1 is -CₙF₂ₙ₊₁,
n is an integer from 1 to 20,
R2 is hydrogen; deuterium; a halogen group; an ester group; or a substituted or unsubstituted straight-chained or branched alkyl group having 1 to 10 carbon atoms,
r2 is an integer from 1 to 4,
when r2 is 2 or higher, two or more R2's are the same as or different from each other.

Throughout the specification of the present application, the term "combination thereof" included in the Markush type expression means a mixture or combination of one or more selected from the group consisting of constituent elements described in the Markush type expression, and means including one or more selected from the group consisting of the above-described constituent elements.

Examples of the substituents in the present specification will be described below, but are not limited thereto.

In the present specification, 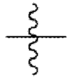 means a moiety to be linked.

The term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more are substituted, the two or more substituents may be the same as or different from each other.

In the present specification, the term "substituted or unsubstituted" means being substituted with one or more substituents selected from the group consisting of deuterium; a halogen group; a hydroxyl group; an ester group; an alkyl group; an alkoxy group; and an aryl group, being substituted with a substituent to which two or more substituents among the exemplified substituents are linked, or having no substituent.

In the present specification, the fact that two or more substituents are linked indicates that hydrogen of any one substituent is linked to another substituent. For example, when two substituents are linked to each other, a phenyl group and a naphthyl group may be linked to each other to become a substituent of 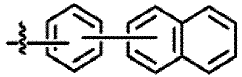 or 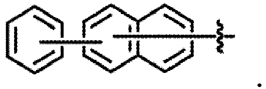 Further, the case where three substituents are linked to one another includes not only a case where (Substituent 1)-(Substituent 2)-(Substituent 3) are consecutively linked to one another, but also a case where (Substituent 2) and (Substituent 3) are linked to (Substituent 1). For example, a phenyl group, a naphthyl group, and an isopropyl group may be linked to one another to become a substituent of 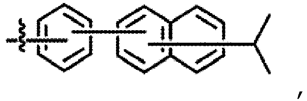 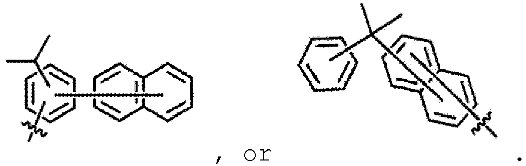 The above-described definition also applies equally to the case where four or more substituents are linked to one another.

In the present specification, examples of a halogen group include a fluoro group, a chloro group, a bromo group or an iodo group.

In the present specification, for an ester group, the oxygen of the ester group may be substituted with a straight-chained alkyl group having 1 to 30 carbon atoms, a branched alkyl group having 3 to 30 carbon atoms, or a cyclic alkyl group having 3 to 30 carbon atoms, or an aryl group having 6 to 30 carbon atoms. Specifically, the ester group may be any one of the following structural formulae, but is not limited thereto.

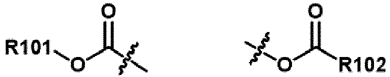

In the structures, R101 and R102 are the same as or different from each other, and are each independently a straight-chained or branched alkyl group having 1 to 10 carbon atoms, which is unsubstituted or substituted with a monocyclic or polycyclic aryl group having 6 to 30 carbon atoms; or a monocyclic or polycyclic aryl group having 6 to 30 carbon atoms.

In the present specification, the alkyl group may be straight-chained or branched, and the number of carbon atoms thereof is not particularly limited, but is preferably 1 to 30. Specific examples thereof include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group, and the like, but are not limited thereto.

In the present specification, the alkoxy group may be straight-chained, branched, or cyclic. The number of carbon atoms of the alkoxy group is not particularly limited, but is preferably 1 to 30. Specific examples thereof include a methoxy group, an ethoxy group, an n-propoxy group, an isopropoxy group, an n-butoxy group, an isobutoxy group, a tert-butoxy group, a sec-butoxy group, an n-pentyloxy group, an neopentyloxy group, an isopentyloxy group, an n-hexyloxy group, a 3,3-dimethylbutyloxy group, a 2-ethylbutyloxy group, an n-octyloxy group, an n-nonyloxy group, an n-decyloxy group, a benzyloxy group, a p-methylbenzyloxy group, and the like, but are not limited thereto.

According to an exemplary embodiment of the present specification, n is an integer from 4 to 12.

According to an exemplary embodiment of the present specification, R1 is -C₄F₉, -C₆F₁₃, -C_{B}F₁₇, or -C₁₂F₂₅.

According to an exemplary embodiment of the present specification, when n is within the above range, compounds with various molecular weights may be prepared because the chain length of a perfluoroalkyl group is easily adjusted. In addition, when a perfluoroalkyl group is included, it is possible to exhibit effects of achieving low refractive index, low relative permittivity, low surface energy and low dissipation factor. Specifically, although compounds containing a perfluoroalkyl group in the related art exhibit low permittivity and low refractive properties, compatibility is not easily exhibited. However, when R1 of Chemical Formula 1 of the present invention is used, it is possible to exhibit the effects of achieving effective low refractive index, low relative permittivity, low surface energy and low dissipation factor while maintaining compatibility.

The compound of Chemical Formula 1 according to an exemplary embodiment of the present specification is a single molecule including both a perfluoroalkyl group and a vinyl group, and may be used for various applications such as electronic materials.

Furthermore, since the compound of Chemical Formula 1 according to an exemplary embodiment of the present specification includes both a perfluoroalkyl group and a vinyl group, the compound may be used as a monomer for preparing a polymer, and may be used for preparing a polymer including a perfluoroalkyl group.

According to an exemplary embodiment of the present specification, R2 is hydrogen; an ester group; or a substituted or unsubstituted straight-chained or branched alkyl group having 1 to 10 carbon atoms.

According to an exemplary embodiment of the present specification, R2 is hydrogen; an ester group; or a straight-chained or branched alkyl group having 1 to 10 carbon atoms, which is unsubstituted or substituted with one or more of a hydroxyl group, a straight-chained or branched alkoxy group having 1 to 10 carbon atoms, and a combination thereof.

According to an exemplary embodiment of the present specification, R2 is hydrogen; an ester group; a methyl group substituted with a hydroxyl group; or a methyl group substituted with a straight-chained or branched alkoxy group having 1 to 10 carbon atoms substituted with a straight-chained or branched alkoxy group having 1 to 10 carbon atoms.

According to an exemplary embodiment of the present specification, R2 is hydrogen; an ester group; a methyl group substituted with a hydroxyl group; or a methyl group substituted with an ethoxy group substituted with a methoxy group.

According to an exemplary embodiment of the present specification, in R2, the ester group is

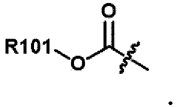

R101 is a straight-chained or branched alkyl group having 1 to 10 carbon atoms, which is unsubstituted or substituted with a monocyclic or polycyclic aryl group having 6 to 30 carbon atoms; or a monocyclic or polycyclic aryl group having 6 to 30 carbon atoms.

According to an exemplary embodiment of the present specification, R101 is a methyl group; an ethyl group; a tert-butyl group; a methyl group substituted with a phenyl group; or an n-decyl group.

According to an exemplary embodiment of the present specification, r2 is 4.

According to an exemplary embodiment of the present specification, r2 is 2.

According to an exemplary embodiment of the present specification, r2 is 1.

According to an exemplary embodiment of the present specification, Chemical Formula 1 is any one selected from the following compounds.

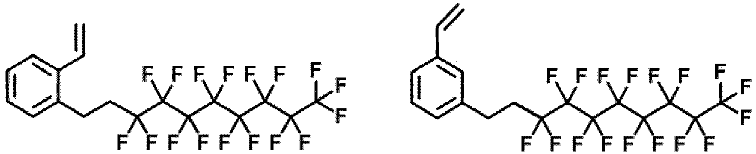

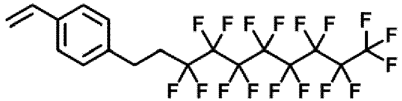

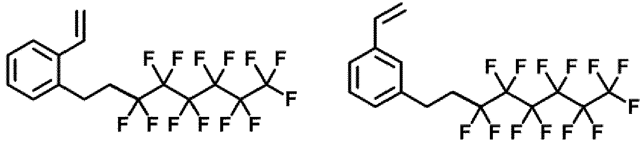

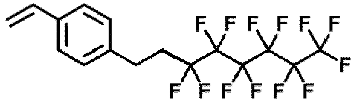

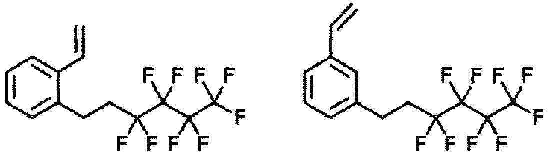

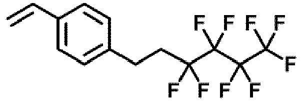

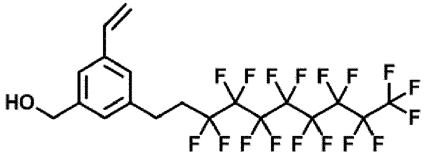

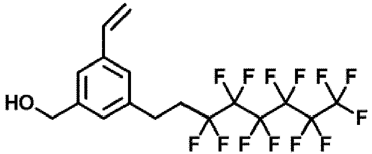

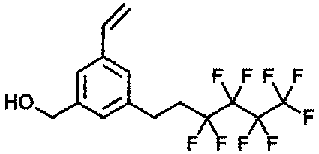

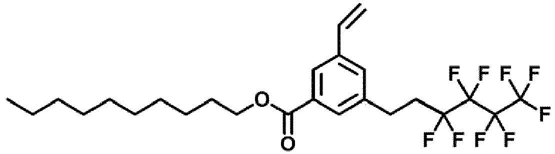

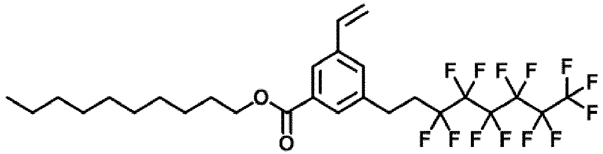

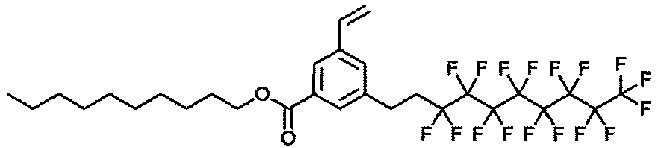

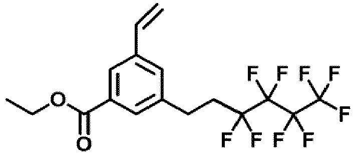

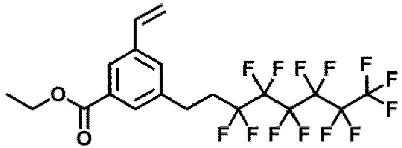

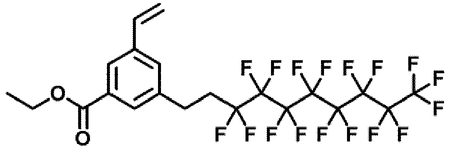

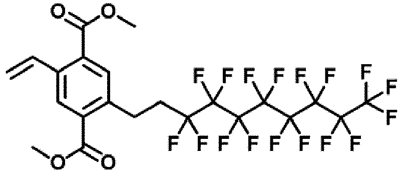

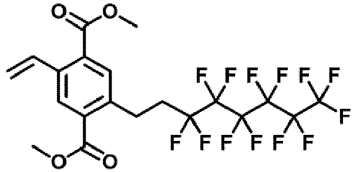

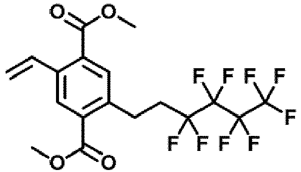

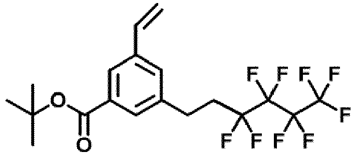

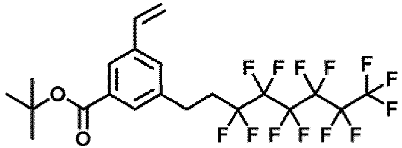

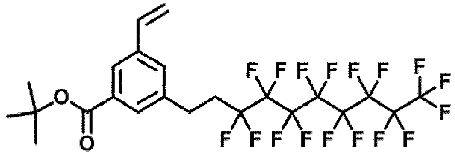

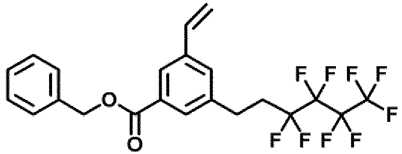

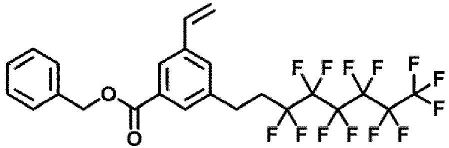

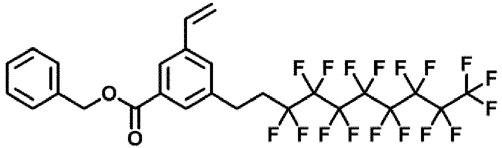

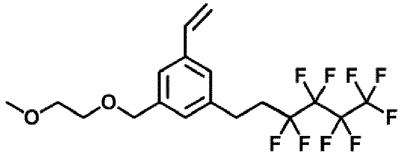

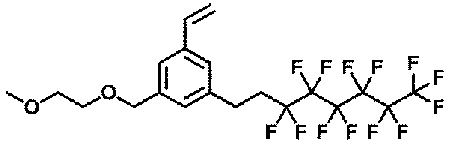

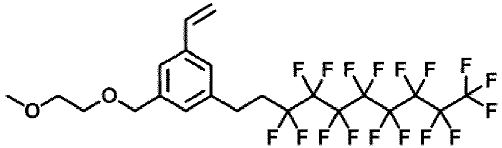

According to an exemplary embodiment of the present specification, Chemical Formula 1 includes a mixture of the compounds of Chemical Formula 1.

Specifically, the mixture of the compounds of Chemical Formula 1 means that two or more of the compounds of Chemical Formula 1 are included, and the structures thereof may be the same as or different from each other.

An exemplary embodiment of the present specification provides a method for preparing a compound of the following Chemical Formula 1, the method including: (s1) introducing the following Chemical Formula A, CₙF₂ₙ₊₁X, trialkyltin hydride and a solvent and stirring the resulting mixture under nitrogen gas; and (s2) adding a radical initiator thereto and stirring the resulting mixture.

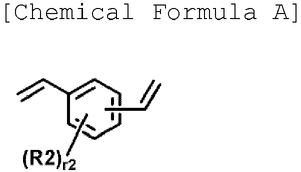

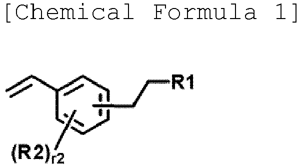

In Step (s1), Chemical Formula A and Chemical Formula 1,
R1 is -CₙF₂ₙ₊₁,
n is an integer from 1 to 20,
R2 is hydrogen; deuterium; a halogen group; an ester group; or a substituted or unsubstituted straight-chained or branched alkyl group having 1 to 10 carbon atoms,
r2 is an integer from 1 to 4,
when r2 is 2 or higher, two or more R2's are the same as or different from each other, and
X is a halogen group.

According to an exemplary embodiment of the present specification, Chemical Formula 1 includes a mixture of the compounds of Chemical Formula 1.

Specifically, the mixture of the compounds of Chemical Formula 1 means that two or more of the compounds of Chemical Formula 1 are included, and the structures thereof may be the same as or different from each other.

According to an exemplary embodiment of the present specification, the reaction temperature in Steps (s1) and (s2) is 20°C to 40°C, preferably 25°C to 35°C, and more preferably 30°C.

When the reaction is performed at the reaction temperature, reaction conditions are mild, the polymerization of the styrene of Chemical Formula A is suppressed, so that side reactions are reduced, and the yield of Chemical Formula 1, which is a final target product, is increased.

According to an exemplary embodiment of the present specification, the radical initiator is an azo initiator.

According to an exemplary embodiment of the present specification, the radical initiator is any one or more selected among azobisisobutyronitrile (AIBN), 2,2'-azobis-2,4-dimethylvaleronitrile, 2,2'-azobis(4-methoxy-2,4-dimethylvaleronitrile), benzoyl peroxide (BPO) or di-tert-butyl peroxide (DTBP).

Preferably, the radical initiator is 2,2'-azobis(4-methoxy-2,4-dimethylvaleronitrile) .

When the radical initiator is used, the radical reaction can be initiated at a low temperature, so that there is an advantage in that the reaction can be performed at the reaction temperature and under mild reaction conditions.

According to an exemplary embodiment of the present specification, the trialkyltin hydride is tributyltin hydride.

The trialkyltin hydride is a hydrogen donor with low binding energy, induces hydrogen atom transfer (HAT) to radicals generated from a small amount of radical initiator, and induces the introduction of a perfluoroalkyl group into Chemical Formula A through an atom transfer radical addition (ATRA) reaction, and the mechanism of the atom transfer radical addition (ATRA) reaction is as shown in FIG. 1.

According to an exemplary embodiment of the present specification, the solvent is an organic solvent.

According to an exemplary embodiment of the present specification, the solvent is selected among hexane, heptane, toluene, benzene, dichloromethane, dichloroethane, trichloroethane, chloroform, dichloroform, nitromethane, dibromomethane, cyclopentanone, cyclohexanone, fluorobenzene, bromobenzene, chlorobenzene, xylene, mesitylene, ethylacetate or any mixture thereof, but is not limited thereto, and an organic solvent used in the related art may be used.

According to an exemplary embodiment of the present specification, X is an iodine (I) group.

According to an exemplary embodiment of the present specification, a step of obtaining the compound of Chemical Formula 1 is further included after Step (s2).

The obtaining of the compound of Chemical Formula 1 according to an exemplary embodiment of the present specification includes:
(s31) a quench and layer separation step;
(s32) a drying step;
(s33) a filtration step;
(s34) a concentration step; and
(s35) an obtaining step.

According to an exemplary embodiment of the present specification, Step (s31) (quench and layer separation step) is a step of terminating the reaction by adding a reaction termination material, and separating the layers.

According to an exemplary embodiment of the present specification, Step (s31) is a step of diluting a reactant by adding the solvent used in Step (s1), terminating the reaction by adding a reaction termination material, and separating layers.

According to an exemplary embodiment of the present specification, as the reaction termination material of Step (s31), distilled water, an aqueous ammonium chloride solution, an aqueous sodium hydrogen carbonate solution, an aqueous KF (saturated) solution, an aqueous HCl solution, an aqueous NaCl (saturated) solution, dichloromethane, ethyl acetate, and the like may be used, but the reaction termination material is not limited thereto. Preferably, as the reaction termination material, an aqueous ammonium chloride solution, an aqueous sodium hydrogen carbonate solution, an aqueous NaCl solution, and ethyl acetate are used.

In Step (s31), the order in which the reaction termination and layer separation are performed is not limited to a specific order. As an exemplary embodiment, reaction termination and layer separation may be simultaneously performed, and as another exemplary embodiment, the reaction is terminated by adding a reaction termination material, and then layers may be separated. However, the progression order is not limited to the above-described example.

According to an exemplary embodiment of the present specification, Step (s32) (drying step) is performed by adding a desiccant to any one layer of the material layer-separated in Step (s31). For example, when dichloromethane exemplified above is used as the reaction termination material, Step (s32) is performed by adding a desiccant to a dichloromethane layer which is a lower layer, that is, an organic layer. Examples of the desiccant include magnesium sulfate, sodium sulfate, and the like, but are not limited thereto. According to a preferred exemplary embodiment, the desiccant is magnesium sulfate.

In the present specification, the desiccant is added in a sufficient amount required for typical drying in the art.

According to an exemplary embodiment of the present specification, Step (s33)(filtration step) may be performed by a method known in the art. As a representative example, it is possible to use a method of inhaling air using an inhaler and filtering impurities including a desiccant, and the like, which are separated from a target product with a filter, or a method of removing other impurities using a solvent that does not dissolve a target product, but dissolves other impurities by utilizing the difference in solubility in a specific solvent.

According to an exemplary embodiment of the present specification, Step (s34)(concentration step) may be performed by a method known in the art. The step may be performed by evaporating the solvent using a vacuum rotary evaporator as a representative example, but the representative example is not limited thereto.

According to an exemplary embodiment of the present specification, additional filtration and drying steps may be added after Step (s34).

It is possible to include a drying step before the obtaining step (s35), and the drying step is a step of drying Chemical Formula 1. The drying step is different from Step (s32) and may be performed by a method known in the art, representative examples thereof include vacuum oven drying, spray drying, flash drying, and the like, and preferably, the compound of Chemical Formula 1 may be dried by vacuum oven drying.

According to an exemplary embodiment of the present specification, Step (s35) (obtaining step) is a step of obtaining the compound of Chemical Formula 1 which is concentrated (dried) in Step (s34).

According to an exemplary embodiment of the present specification, in the obtaining step, Steps (s32) to (s33) may be performed three or more times, if necessary.

According to an exemplary embodiment of the present specification, provided is a mixture including two or more of the compounds.

According to another exemplary embodiment of the present specification, two or more compounds included in the mixture are the same as or different from each other. That is, it means that the two or more compounds are the same as or different from each other while having the structure of Chemical Formula 1.

According to still another exemplary embodiment of the present specification, the mixture may further include a compound different from the compound of Chemical Formula 1.

According to an exemplary embodiment of the present specification, provided is a single molecule derived from the compound.

In this specification, for example, the above-mentioned "single molecule derived from the compound of Chemical Formula 1" may mean a single molecule in which the vinyl group of the compound of Chemical Formula 1 forms a radical and an additional substituent is introduced, or the compound of Chemical Formula 1 itself.

According to an exemplary embodiment of the present specification, provided is a polymer including a monomer derived from the compound.

Those skilled in the art will understand the term "monomer" described in the present specification as a state in which a compound is polymerized to be linked to the main chain of the polymer.

In the present specification, for example, the "monomer derived from the compound of Chemical Formula 1" is a repeating unit that constitutes the main chain in the polymer, and means that a vinyl group of the compound of Chemical Formula 1 may form a radical to become a monomer, and a monomer or end group which makes up the main chain of other polymers may be introduced.

According to an exemplary embodiment of the present specification, the polymer may further include an additional monomer, and the additional monomer is not limited.

According to an exemplary embodiment of the present specification, the polymer may be an alternating polymer or a random polymer, but is not limited thereto.

Further, in the present specification, even when a monomer included in the polymer is mentioned, the monomer is not limited to including only the mentioned monomer, and other monomers in addition to the aforementioned monomer may additionally be included as co-monomers within a range not departing from the object of the present invention.

According to an exemplary embodiment of the present specification, provided is a resin composition including the compound, and the single molecule and polymer derived therefrom.

A compound according to an exemplary embodiment of the present specification, and a single molecule and a polymer derived therefrom are used as an electronic material, an organic insulating material, and/or a substrate material, but are not limited thereto.

A compound according to an exemplary embodiment of the present specification, and a single molecule and a polymer derived therefrom may be used as a semiconductor copper clad laminate (CCL) material. Specifically, representative materials used in semiconductor CCL materials include a plurality of monomers and polymers having a styrene group, monomers and polymers having a bismaleimide group, and the like.

The bismaleimide group has a structure similar to that of maleic anhydride, and actually, it is generally known that a styrene group very effectively forms an alternating copolymer when polymerized with a bismaleimide group. This is because the transition state in the form of non-bonded resonance including electron transfer is stabilized by a polar effect charge transfer interaction.

Therefore, it is expected that the compound of Chemical Formula 1, which includes a perfluoroalkyl group having relative permittivity properties, can more effectively induce the thermoset networks generated during curing than compounds in the related art. An additional new additive does not need to be used for this reason, and it is possible to effectively lower the permittivity of existing insulating layers without phase separation with existing materials.

### [Mode for Invention]

Hereinafter, the present specification will be described in detail with reference to Examples for specifically describing the present specification. However, the Examples according to the present specification may be modified in various forms, and it is not interpreted that the scope of the present specification is limited to the Examples described below in detail. The Examples of the present specification are provided to explain the present specification more completely to a person with ordinary skill in the art.

### Example 1.

After 20.8 g of divinylbenzene, 6.9 mL of nonafluoro-1-iodobutane, and 10.8 mL of tributyltin hydride were dissolved in 40 mL of dichloromethane in a 100 mL two-neck round-bottom flask, the resulting mixture was bubbled with nitrogen at 30°C for 30 minutes. After 0.37 g of 2,2'-azobis(4-methoxy-2,4-dimethylvaleronitrile) (V-70) was added thereto, the resulting mixture was stirred for 15 hours. Thereafter, the mixture was diluted with 100 mL of dichloromethane, and the organic layer was washed with 100 mL of a KF saturated solution, 100 mL of a NaCl saturated solution and 100 mL of water, and then dried over 10 g of MgSO₄. After filtration and evaporation, 7.9 g of the following Compound 1 was obtained.

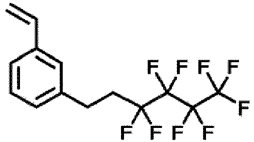

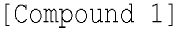

### Example 2.

After 26.0 g of divinylbenzene, 10.7 mL of heptadecafluoro-n-octyl iodide, and 10.8 mL of tributyltin hydride were dissolved in 40 mL of dichloromethane in a 100 mL two-neck round-bottom flask, the resulting mixture was bubbled with nitrogen at 30°C for 30 minutes. After 0.37 g of 2,2'-azobis(4-methoxy-2,4-dimethylvaleronitrile) (V-70) was added thereto, the resulting mixture was stirred for 15 hours. Thereafter, the mixture was diluted with 100 mL of dichloromethane, and the organic layer was washed with 100 mL of a KF saturated solution, 100 mL of a NaCl saturated solution and 100 mL of water, and then dried over 10 g of MgSO₄. After filtration and evaporation, 15.4 g of the following Compound 2 was obtained.

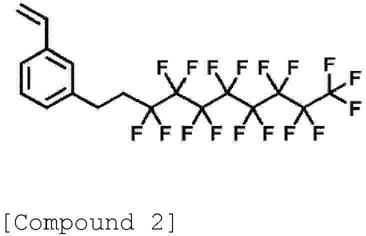

The following Table 1 describes the amounts of vinyl and perfluoroalkyl groups of Compounds 1 and 2 prepared from Examples 1 and 2.

**[Table 1]**

| (by ¹H NMR, mmol/g) | Vinyl group per g | Perfluoroalkyl group per g |
|---|---|---|
| Example 1 | 2.24 | 4.01 |
| Example 2 | 0.68 | 3.06 |

According to Table 1, the compound of Chemical Formula 1 includes both a vinyl group and a perfluoroalkyl group in one molecule, and thus, may be used as a monomer for preparing a polymer, and through this, it can be seen that the compound of Chemical Formula 1 may be used for preparing a polymer including a perfluoroalkyl group.

## Claims

1. A compound of the following Chemical Formula 1: 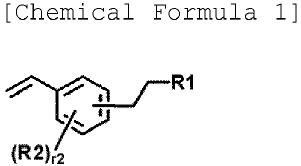 wherein, in Chemical Formula 1,
R1 is -CₙF_{2n+1,}
n is an integer from 1 to 20,
R2 is hydrogen; deuterium; a halogen group; an ester group; or a substituted or unsubstituted straight-chained or branched alkyl group having 1 to 10 carbon atoms,
r2 is an integer from 1 to 4, and
when r2 is 2 or higher, two or more R2's are the same as or different from each other.

2. The compound of claim 1, wherein R1 is -C₄F₉, -C₆F₁₃, -C₈F₁₇, or -C₁₂F₂₅.

3. The compound of claim 1, wherein R2 is hydrogen; an ester group; or a straight-chained or branched alkyl group having 1 to 10 carbon atoms, which is unsubstituted or substituted with one or more of a hydroxyl group, a straight-chained or branched alkoxy group having 1 to 10 carbon atoms, and a combination thereof.

4. The compound of claim 1, wherein Chemical Formula 1 is any one selected from the following compounds:

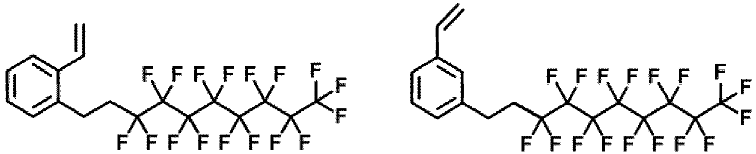

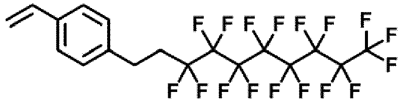

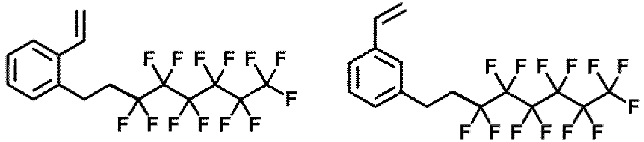

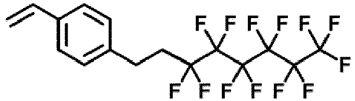

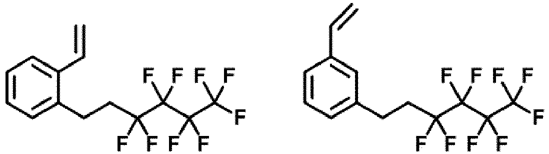

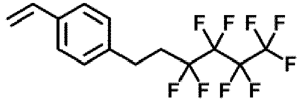

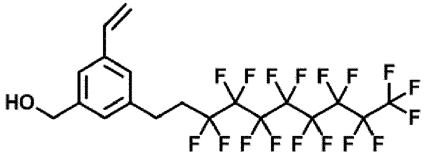

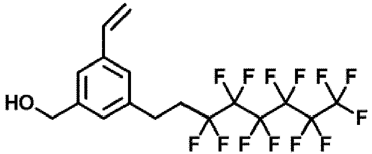

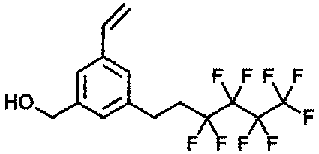

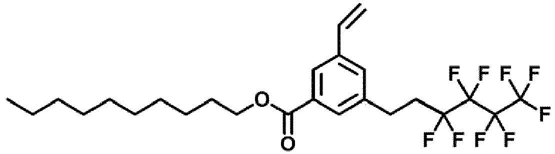

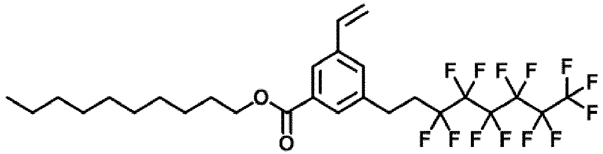

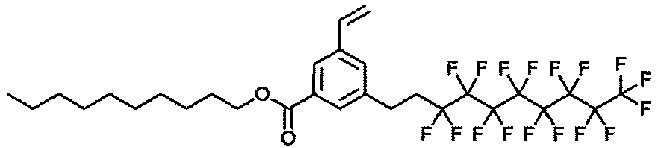

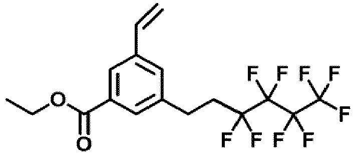

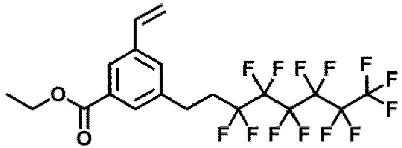

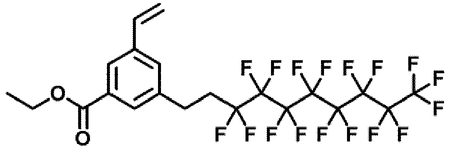

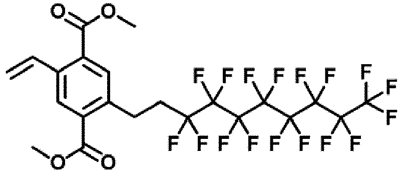

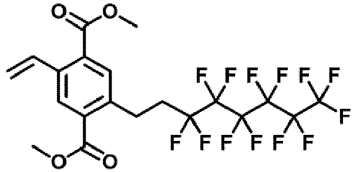

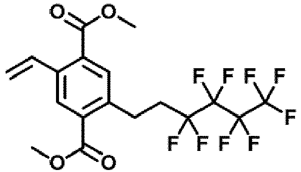

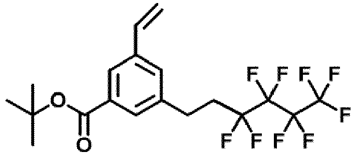

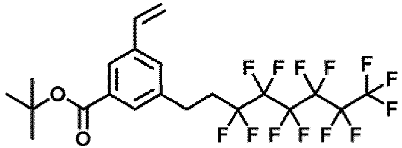

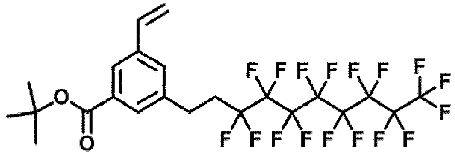

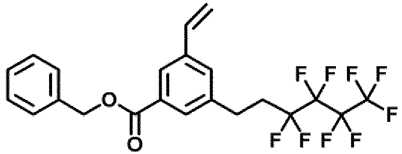

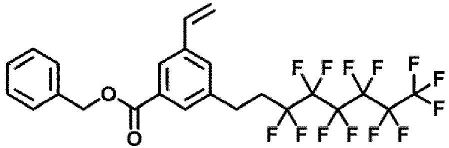

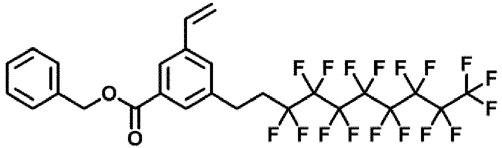

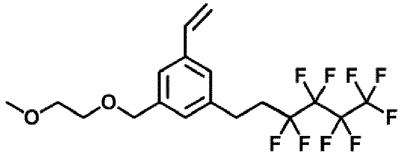

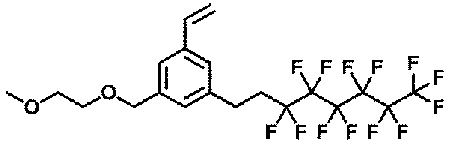

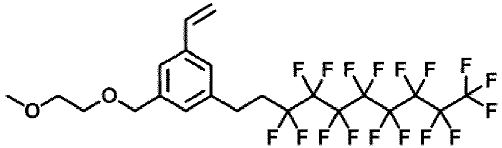

5. A method for preparing a compound of the following Chemical Formula 1, the method including:
(s1) introducing the following Chemical Formula A, CₙF₂ₙ₊₁X, trialkyltin hydride and a solvent and stirring the resulting mixture under nitrogen gas; and
(s2) adding a radical initiator thereto and stirring the resulting mixture: 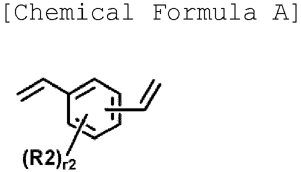 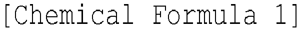 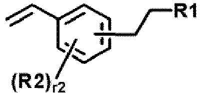
wherein, in Step (s1), Chemical Formula A and Chemical Formula 1,
R1 is -CₙF_{2n+1,}
n is an integer from 1 to 20,
R2 is hydrogen; deuterium; a halogen group; an ester group; or a substituted or unsubstituted straight-chained or branched alkyl group having 1 to 10 carbon atoms,
r2 is an integer from 1 to 4,
when r2 is 2 or higher, two or more R2's are the same as or different from each other, and
X is a halogen group.

6. A mixture comprising two or more of the compounds of any one of claims 1 to 4.

7. A single molecule derived from the compound of any one of claims 1 to 4.

8. A polymer comprising a monomer derived from the compound of any one of claims 1 to 4.

9. The polymer of claim 8, wherein the polymer further comprises an additional monomer.
